(19) Europäisches Patentamt
European Patent Office
Office européen des brevets

(11) Veröffentlichungsnummer: **0 259 698 A2**

(12) **EUROPÄISCHE PATENTANMELDUNG**

(21) Anmeldenummer: **87112377.4**

(22) Anmeldetag: **26.08.87**

(51) Int. Cl.4: **C07H 15/244** , A61K 31/70 , C12P 19/56 , //(C12P19/56,C12R1:54)

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 3093.

Patentansprüche für folgenden Vertragsstaaten: AT + ES + GR.

(30) Priorität: **28.08.86 DE 3629274**

(43) Veröffentlichungstag der Anmeldung: **16.03.88 Patentblatt 88/11**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Zeeck, Axel, Prof. Dr.**
**Brüder-Grimm-Allee 22**
**D-3400 Göttingen(DE)**
Erfinder: **Ciesiolka Thomas**
**Gartemühle**
**D-3400 Göttingen(DE)**
Erfinder: **Rohr, Jürgen, Dr.**
**Valentinsbreite 47**
**D-3400 Göttingen(DE)**
Erfinder: **Zähner, Hans, Prof. Dr.**
**Im Hopfengarten 13**
**D-7400 Tübingen(DE)**
Erfinder: **Drautz, Hannelore, Dr.**
**Tulpenweg 16**
**D-7406 Mössingen(DE)**

(54) **Urdamycin G und dessen Derivate,Verfahren zu ihrer Herstellung und ihre Verwendung.**

(57) Es wurde ein neuer Stoff, Urdamycin G, gefunden, der ebenso wie seine O-Acyl-Derivate antibiotische bzw. Antitumor-Aktivität zeigt.

EP 0 259 698 A2

# Urdamycin G und dessen Derivate, Verfahren zu ihrer Herstellung und ihre Verwendung

In der Deutschen Offenlegungsschrift 3 441 933 werden die Urdamycine A bis F und ihre Aglykone, die entsprechenden Urdamycinone, beschrieben. Die Urdamycine werden durch Fermentation mit einem Mikroorganismenstamm, der aus einer Bodenprobe aus Tansania isoliert wurde, hergestellt. Der Mikroorganismus wurde als Streptomyces fradiae identifiziert und bei der Deutschen Sammlung von Mikroorganismen (DSM) unter der Nummer DSM 3093 hinterlegt. Die entsprechenden Urdamycinone kann man durch saure Hydrolyse oder Methanolyse der Urdamycine erhalten.

Die bekannten Urdamycine und Urdamycinone wirken antibakteriell und tumorhemmend und können daher in Form pharmazeutischer Präparate zur Behandlung von bakteriell verursachten Infektionen oder zur Behandlung bei Tumorerkrankungen an Mensch und Tier verwendet werden.

Man hat beobachtet, daß die Zusammensetzung des Gemisches von Urdamycin A bis F von den Kulturbedingungen des Mikroorganismus abhängig ist. So erhält man bei Verwendung eines Nährmediums, das aus Vollfett-Soja (2 %) und Glucose (2 %) besteht, Urdamycin A als Hauptprodukt (65 %), während unter Zusatz von MES-Puffer (Morpholinoethansulfonsäure) zu diesem Nährmedium die Urdamycin A-Produktion stark zurückgedrängt wird und Urdamycin C und Urdamycin D vorwiegend gebildet werden. Urdamycin E ist das Hauptprodukt (53 %) beim Einsatz eines Fleischextrakt-, Glucose-Nährmediums (je 20 %). Urdamycin B entsteht als Hauptprodukt, wenn Vollfett-Soja (2 %), Glucose (1 %), Stärke (1 %) und $NaHPO_4$ (1 %) als Nährmedium verwendet wurden.

Es wurde nun gefunden, daß Urdamycin G ebenfalls mit Hilfe von Streptomyces fradiae hergestellt werden kann. Die Ausbeuten sind auch hier fermentationsabhängig. Die neue Verbindung und deren Acyl-Derivate sind ebenso wie die bekannten Urdamycine antibakteriell und tumorhemmend. Insbesondere die Acyl-Derivate zeigen sogar eine höhere Aktivität.

Die Erfindung betrifft somit:

1. Die Verbindung der allgemeinen Formel I,

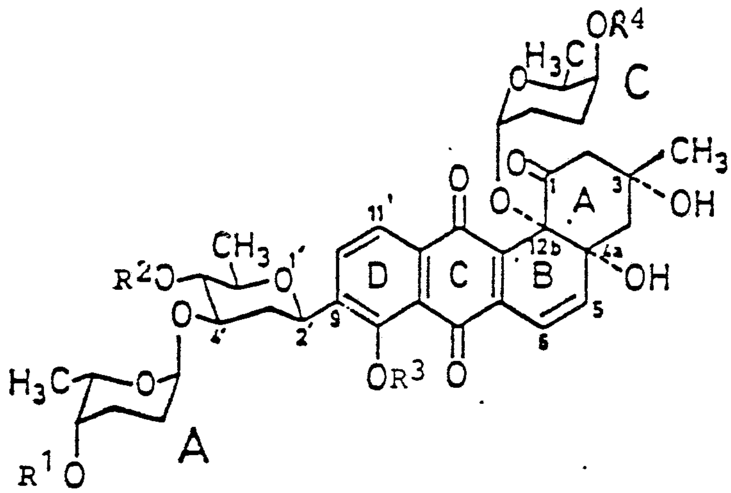

I

in der $R^1$-$R^4$ unabhängig voneinander Wasserstoff oder eine ($C_1$-$C_{18}$)Acylgruppe bedeutet.

2. Ein Verfahren zur Herstellung der Verbindung der allgemeinen Formel I, das dadurch gekennzeichnet ist, daß man Streptomyces fradiae fermentiert, die Verbindung der allgemeinen Formel I aus dem Kulturmedium isoliert und gegebenenfalls acyliert.

3. Die Verwendung der Verbindung der allgemeinen Formel I zur Herstellung von Heilmitteln.

Im folgenden wird die Erfindung, insbesondere mit ihren bevorzugten Ausgestaltungen, erläutert und in den Patentansprüchen definiert.

Steptomyces fradiae ist wie folgt charakterisiert:

Sporenoberfläche: Sm
Sporenmorphologie: Sa
Chromogenität: C-
Luftmycelfarbe: cinnamomeus

Für die Fermentation wird Streptomyces fradiae, insbesondere der Stamm DSM 3093, eingesetzt. Anstelle von DSM 3093 können auch dessen Mutanten und Varianten eingesetzt werden, soweit sie Urdamycin G synthetisieren. Solche Mutanten können in an sich bekannter Weise durch physikalische Mittel, beispielsweise Bestrahlung, wie Ultraviolett-oder Röntgenstrahlen, oder chemische Mutagene, wie beispielsweise Methansulfonsäureethylester (Ethylmethansulfonat, EMS) oder 2-Hydroxy-4-methoxybenzophenon (MOB) erzeugt werden.

Als bevorzugte Kohlenstoffquellen für die aerobe Fermentation eignen sich assimilierbare Kohlenhydrate und Zuckeralkohole, wie Glucose, Lactose oder D-Mannit, sowie kohlenhydrathaltige Naturprodukte, wie Malzextrakt. Als bevorzugte stickstoffhaltige Nährstoffe kommen in Betracht: Aminosäuren, Peptide und Proteine sowie deren Abbauprodukte, wie Peptone oder Tryptone, ferner Fleichextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. Außerdem kann die Nährlösung beispielsweise Chloride, Carbonate, Sulfate oder Phosphate der Alkali-oder Erdalkalimetalle, Eisen, Zink und Mangan als zusätzliche anorganische Salze enthalten. Die Bildung des Urdamycin G verläuft besonders gut in einer Nährlösung, die Vollfett-Soja in Konzentrationen von 0,5 bis 5 %, bevorzugt 2 %, sowie Glucose in Konzentrationen von 0,5 bis 4 %, bevorzugt 2 %, enthält, jeweils bezogen auf das Gewicht der gesamten Nährlösung.

Die Fermentation erfolgt aerob, also beispielsweise submers unter Schütteln oder Rühren in Schüttelkolben oder Fermentern, gegebenenfalls unter Einführen von Luft oder Sauerstoff. Die Fermentation kann in einem Temperaturbereich von etwa 18 bis 40°C, vorzugsweise bei etwa 25 bis 30°C, insbesondere bei 27 bis 28°C erfolgen. Man kultiviert den Mikroorganismus unter den genannten Bedingungen bis die stationäre Phase erreicht ist, etwa 60 bis 80 Stunden, bevorzugt 70 bis 75 Stunden.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1 : 10, überimpft werden. Die Vorkultur erhält man beispielsweise, indem man ein versportes Mycel in eine Nährlösung überimpft und etwa 48 bis 72 Stunden wachsen läßt. Das versporte Mycel kann erhalten werden, indem man den Stamm etwa 7 Tage auf einem festen oder flüssigen Nährboden, beispielsweise Hefe-Malz-Agar, wachsen läßt.

Der Fermentationsverlauf kann anhand des pH-Wertes der Kultur oder des Mycelvolumens, durch Dünnschichtchromatographie oder Ausprüfen der biologischen Aktivität überwacht werden.

Die Isolierung von Urdamycin G aus dem Kulturmedium erfolgt nach bekannten Methoden unter Berücksichtigung der chemischen, physikalischen und biologischen Eigenschaften der Produkte. Zum Testen der Antibiotika-Konzentration im Kulturmedium oder in den einzelnen Isolierungsstufen kann die Dünnschichtchromatographie, beispielsweise auf Kieselgel mit Chloroform/Methanol als Laufmittel verwendet werden, wobei die Menge der gebildeten Antibiotika zweckmäßig mit einer Eichlösung verglichen wird.

Urdamycin G liegt zusammen mit den bekannten Urdamycinen A bis F im Mycel bzw. in der Kulturbrühe vor. Die Antibiotika können aus der unfiltrierten Kulturbrühe mit einem mit Wasser nicht oder nur wenig mischbaren organischen Lösemittel, wie Chloroform oder Ethylacetat, extrahiert werden. Da sie sich jedoch nur zu einem geringen Teil im Mycel befinden, ist es vorteilhaft, die Kulturbrühe vom Mycel zu trennen, beispielsweise durch Zentrifugieren oder Filtrieren, vorzugsweise unter Zugabe von Filterhilfsmitteln.

Das Antibiotikum kann dann aus dem Überstand bzw. Filtrat isoliert werden, zweckmäßig im leicht sauren bis neutralen pH-Bereich, vorzugsweise bei pH 6 bis 7. Dazu verwendet man zweckmäßig mit Wasser wenig oder nicht mischbare organische Lösemittel, insbesondere chlorierte Kohlenwasserstoffe, wie Chloroform oder Methylenchlorid oder Ester wie Ethylacetat. Die farbigen Extrakte werden, gegebenenfalls nach Eindampfen und Aufnehmen in einem polaren organischen Lösemittel, durch Fällen mit einem unpolaren Lösemittel, zweckmäßig einem Kohlenwasserstoff wie Petrolether, von stark lipophilen Bestandteilen befreit, die bis zu etwa 80% des Gesamtextraktes ausmachen können. Aus dem Rückstand kann Urdamycin G durch Chromatographie isoliert werden.

Zur Isolierung des Urdamycin G aus dem entfetteten Rohextrakt wird dieser zweckmäßig über eine Kieselgel-Säule gereinigt, wobei sich als Fleißmittel Gemische aus niedermolekularen Chlorkohlenwasserstoffen und Alkanolen, beispielsweise Chloroform und Methanol im Volumenverhältnis 4 : 1 oder Methylenchlorid und Methanol im Volumenverhältnis 85 : 15, bewährt haben. Die in Form verschiedenfarbiger Zonen adsorbierten Komponenten werden nacheinander in Form verschiedenfarbiger Eluate eluiert. In den beiden Hauptfraktionen ist Urdamycin G (2. Fraktion) und Urdamycin A (3. Fraktion, siehe DOS 3441933) enthalten.

Zur Isolierung des reinen Urdamycin G können die üblichen Verfahrensschritte Verwendung finden, wie Chromatographie, Gelfiltration oder Fällung aus ihren Lösungen in geeigneten organischen Lösemitteln. Besonders bewährt hat sich eine Chromatographie an Kieselgel, wobei als Laufmittel eine Mischung aus einem niederen halogenierten Kohlenwasserstoff und einem niederen Alkanol, beispielsweise Methylenchlorid/Ethanol oder Chloroform/Methanol in einem Volumenverhältnis von beispielsweise 9 : 1 Verwendung findet, gefolgt von einer Gelfiltration an geeigneten Medien wie Hydroxyalkoxypropyldextranen (lipophile (R)SEPHADEX LH-Marken) mit einem niederen Alkanol wie Methanol und anschließendes Ausfällen, beispielsweise durch Eintropfen einer konzentrierten Lösung des Urdamycins in einem polaren organischen Lösemittel, wie Aceton, in ein unpolares Lösemittel, insbesondere einen Kohlenwasserstoff, wie Petrolether oder n-Hexan.

Urdamycin G ist ein oranger, amorpher Feststoff, der gut in Methanol, Aceton, DMSO, Dioxon und Chloroform löslich ist, jedoch nicht in Wasser und Alkanen. Die Substanz ist im festen Zustand wie auch als Lösung im pH-Bereich von 3 bis 9, insbesondere von 5 bis 8, stabil und läßt sich somit in übliche galenische Zubereitungen einarbeiten.

Die antibakterielle Wirkung kommt sowohl gegen gram-positive wie gram-negative Bakterien zur Geltung, wie beispielsweise im Platten-Agardiffusiontest in vitro (10 µl/Testrondell, 6 mm Durchmesser) gezeigt werden kann. Verschiedene Keime. die sich gegenüber einer minimalen Hemmkonzentration im Bereich von > 0,03 bis < 0,3 µl/ml sensitiv zeigen, sind beispielsweise: Achromobacter geminiani, Bacillus brevis, B. subtilis, Arthrobacter aurescens, A. crystallopoiete, Brevibacetrium flavum sowie verschiedene Streptomyceten, darunter z.B. S. antibioticus, S. violaceoruber, S. prasinus, S. lavendulae, glaucescens und S. viridochromogenes.

Die tumorhemmende Wirkung von Urdamycin G konnte an menschlichen und tierischen Tumorzellen in vitro gezeigt werden.

Die Überführung des Urdamycin G in die entsprechenden ($C_1$-$C_{18}$)-Acylverbindungen, bevorzugt ($C_1$-$C_{10}$) Acylverbindungen, insbesondere die Acetylverbindung, erfolgt nach an sich bekannter Weise mit einem Acylierungsmittel, das die gewünschten Acylreste einer organischen Carbonsäure einführt. Dabei verwendet man die entsprechende Carbonsäure oder ein reaktionsfähiges Derivat, insbesondere ein Anhydrid. Die Acylierung kann in Gegenwart geeigneter Kondensationsmittel, bei Verwendung von freien Carbonsäuren, z.B. in Gegenwart von Carbodiimidverbindungen, wie Dicyclohexylcarbodiimid, oder Carbonylverbindungen, wie Diimidazolylcarbonyl, und bei Verwendung von reaktionsfähigen Carbonsäurederivaten, z.B. in Gegenwart von basischen Mitteln, wie Triniederalkylamin, z.B. Triethylamin, oder heterocyclischen Basen, z.B. Pyridin oder 4-Dimethylaminopyridin, oder basischen Salzen, z.B. wasserfreies Natriumacetat, durchgeführt werden. Die Acylierungsreaktion kann in Abwesenheit oder in Gegenwart eines Lösungsmittels oder Lösungsmittelgemisches unter Kühlen, bei Raumtemperatur oder unter Erwärmen und, wenn notwendig, in einem geschlossenen Gefäß und/oder in einer Inertgas-, z.B. Stickstoffatmosphäre, durchgeführt werden. Geeignete Lösungsmittel sind beispielsweise einfache oder substituierte, z.B. chlorierte, aliphatische, cycloaliphatische oder aromatische Kohlenwasserstoffe, wobei man geeignete Veresterungsreagentien, wie Acetanhydrid, aber auch geeignete Basen, wie Pyridin, als Verdünnungsmittel verwenden kann.

Bevorzugt wird die Reaktion jedoch mit einem Anhydrid, insbesondere Acetanhydrid, in Gegenwart von Pyridin oder Natriumacetat durchgeführt. Die Reaktionstemperaturen und die Reaktionszeiten liegen zwischen -10 bis +100 °C und 2 Minuten bis 48 Stunden, bevorzugt zwischen 20 bis 30 °C und 8 Minuten bis 16 Stunden. Das Verhältnis Anhydrid zu Base liegt im Bereich von 20:1 bis 1:1, bevorzugt bei 2:1. Die Konzentration von Urdamycin G im Reaktionsansatz liegt zwischen 0,05 bis 10 %, bevorzugt bei 0,1 bis 1 %.

Nach Beendigung der Reaktion kann das Reaktionsprodukt durch Extraktion isoliert werden. Zur weiteren Reinigung wird es dann beispielsweise an Kieselgel chromatographiert.

Alternativ können Mono-bis Triacylverbindungen durch basische Acylspaltung von 5',4A,4C,8-Tetra-o-acyl-urdamycin G hergestellt werden. Verwendung finden Basen, wie die Alkali-oder Erdalkalihydroxide oder Alkalicarbonate, in wäßrig/alkoholischer Lösung bei -10° bis +100 °C. Die Acylspaltung wird bevorzugt mit einer gesättigten Natriumcarbonatlösung in wäßrig/methanolischer Lösung bei 25 °C durchgeführt. Die anschließende Aufarbeitung erfolgt wie oben erwähnt.

Die genannten Acylgruppen leiten sich von organischen Carbonsäuren ab. Diese enthalten geradkettige, verzweigte oder cyclische aliphatische, aliphatisch-aromatische oder aromatische Kohlenwasserstoffreste, die ihrerseits unsubstituiert oder durch Halogen, z.B. Chlor oder Brom, veresterte oder veretherte Hydroxygruppen substituierte sind.

Die so hergestellten Verbindungen zeigen ebenfalls tumorhemmende Wirkung.

Im folgenden wird die Erfindung anhand von Beispielen detailliert erläutert: Prozentangaben beziehen sich, wenn nicht anders angegeben, auf das Gewicht.

Beispiele:

1. a) Herstellung einer Sporensuspension des Produzentenstammes:

100 ml Nährlösung (4 g Hefeextrakt, 10 g Malzextrakt, 4 g Glucose, 1 l Leitungswasser, pH-Wert vor dem Sterilisieren 7,3) in einem 500 ml Erlenmeyerkolben werden mit dem Stamm DSM 3093 beimpft und 72 Stunden bei 27°C und 120 UpM auf der rotierenden Schüttelmaschine inkubiert. Anschließend werden 20 ml Kulturflüssigkeit in einem 500 ml Erlenmeyerkolben mit dem Nährboden der oben genannten Zusammensetzung, dem 20 g Agar/l zur Verfestigungen zugegeben wurde, gleichmäßig verteilt und dekantiert. Die Kulturen werden 10 bis 14 Tage bei 27°C inkubiert. Die nach dieser Zeit entstandenen Sporen eines Kolbens werden mit 500 ml entionisiertem Wasser, das einen Tropfen eines handelsüblichen nichtionischen Tensids enthält, abgeschwemmt, sofort weiterverwendet oder bei -22°C aufbewahrt.

b) Herstellung einer Kultur bzw. Vorkultur des Produzentenstammes im Erlenmeyerkolben.

Ein 500 ml Erlenmeyerkolben mit 100 ml einer Nährlösung der Zusammensetzung 2 % Fleischmehl, 10 % Malzextrakt, 1 % Calciumcarbonat und Wasser ad 100 % (pH 7,2 vor dem Autoklavieren) wird mit einer auf einem Schrägröhrchen gezogenen Kultur oder mit 0,2 ml Sporensuspension angeimpft und auf einer Schüttelmaschine bei 120 UpM und 27°C inkubiert. Die maximale Antibiotikumproduktion ist nach 72 Stunden erreicht. Zum Animpfen von 10 und 100 l Fermentern genügt eine 48 Stunden alte Submerskultur (5 %) aus der gleichen Nährlösung.

2. Herstellung von Urdamycin G

Ein 10 l Fermenter wird unter folgenden Bedingungen betrieben:
Nährmedium: Vollfett-Soja 2 %
Glucose 2 %
pH 7,2
Inkubationszeit: 72 Stunden
Inkubationstemperatur: 27°C
Rührergeschwindigkeit: 250 UpM
Belüftung: 4 l Luft/min.

Durch wiederholte Zugabe weniger Tropfen ethanolischer Polyollösung kann die Schaumentwicklung unterdrückt werden. Das Produktionsmaximum wird nach ca. 70 Stunden (pH = 5,3) erreicht. Die Ausbeuten liegen bei ca. 40 mg/l Urdamycin G.

3. Isolierung von Urdamycin G

Nach der Fermentation von DSM 3093 wird die Kulturbrühe unter Zusatz von 2 % Celite als Filterhilfsmittel filtriert. Das Mycel wird mit Aceton extrahiert, die organische Phase eingedampft und der wäßrige Rückstand mit Ethylacetat extrahiert. Das Kulturfiltrat wird ebenfalls bei pH 7 erschöpfend mit Ethylacetat extrahiert. Dieser Extrakt wird mit dem des Mycels vereinigt, getrocknet und eingedampft. Der ölige Rückstand wird mit reichlich Petrolether übergossen. Der ausgefallene Niederschlag wird abzentrifugiert und getrocknet. Das Rohprodukt wird an einer Kieselgelsäule (Kieselgel 60, Macherey-Nagel) mit Methylchlorid/Methanol (85:15; v:v) chromatographiert. In Fraktion 2 (gelbrot) des Eluats war Urdamycin G angereichert. Es wurde an Sephadex LH 20 in Methanol nachgereinigt.

4. Charakterisierung von Urdamycin G

Das gereinigte Urdamycin G wird in wenig Aceton gelöst und durch Eintropfen dieser Lösung in den 20-fachen Überschuß n-Hexan ausgefällt. Der so erhaltene orange Feststoff zersetzt sich bei 141°C und hat die folgenden Eigenschaften:

Dünnschichtchromatographie:(Kieselgel SilG-25, UV 254 + 366)
Chloroform/Methanol (85 : 15; v:v): Rf 0,44
Chloroform/Methanol (9 : 1; v:v): Rf 0,30
neg.-FAB-MS: m/e = 714 (M; 12 %); 664 (4 %); 654 (5 %); 281 (54 %); 279 (46 %) entsprechend $C_{37}H_{46}O_{14}$ (714,77)
IR (KBr):
3420; 2958 sh; 2910; 2840; 1718; 1650 sh; 1645 sh; 1630; 1615; 1555 $cm^{-1}$,
UV (Methanol):
$\lambda_{max}(\epsilon)$ = 426,5 (4060); 319,5 (2990) nm
UV (Methanol/HCl):
$\lambda_{max}(\epsilon)$ = 430 (3950); 317 (2900) nm
UV (Methanol/NaOH):
$\lambda_{max}(\epsilon)$ = 577,2 (3800); 404 (1650); 324 (6040) nm
$^1H$-NMR (200 MHz, $CDCl_3$):

$\delta$ = 0,58 (d, J = 6 Hz, 5C-$CH_3$); 1,24 (s, 3-$CH_3$); 1,24 (d, J = 6,5 Hz, 5A-$CH_3$); ca. 1,30 (verd., 3'-$H_a$); 1,43 (d, J = 6 Hz, 6'-$CH_3$); 1,4-2,2 (komplex, 8H, 3A-$H_2$, 2A-$H_2$, 3C-$H_2$, 2C-$CH_2$); 1,84 (d, J = 15 Hz, 4-$H_a$); 2,16 (dd, J = 15; 2 Hz, 4-$H_e$); 2,46 (ddd, J = 13;5;2 Hz, 3'-$H_e$); 2,52 (d. J = 12,5; 2-$H_a$); 2,79 (dd, J = 12,5;2,5 Hz, 2-$H_e$ ); 3,19 (dd, J = 9;9 Hz, 5'-H); 3,4-3,6 (komplex, 2H, 4C-H, 6'-H); 3,70 (s, breit, 4A-H); 3,72 (verd., 5C-H); 3,73 (m, 4'.H); 4,18 (dq, J = 6,5;2 Hz; 5A-H); 4,88 (dd, J = 11,5; 2 Hz, 2'-H); 5,01 (o, J = 3x2 Hz, 1A-H); 5,40 (s, breit, 1C-H); 6,41 (d, J = 10 Hz 5-H); 6,89 (d, J = 10 Hz, 6-H); 7,67 (d, J = 8;0,8 Hz, 11-H); 7,93 (d, J = 8;0,8 Hz, 10-H); 12,33 (s, breit, OH) * ppm

CD (Methanol):
$\lambda_{extr.}(\theta^{20}x10^{-3})$ = 401 (-9,2); 329 (21,2); 289 (10,0); 263 (-4,7); 235 (20,0) nm
$[\alpha]_D^{20}$ (c = 0,1, Methanol): +36°

5. Herstellung von 5',4A,4C,8-Tetra-O-acetyl-urdamycin G

20 mg Urdamycin G werden in 12 ml Acetanhydrid/Pyridin Gemisch (2:1, v:v) 5,5 Stunden bei Raumtemperatur gerührt. Man gießt auf Eis und extrahiert 3 mal mit je 50 ml Chloroform. Man engt die organische Phase im Vakuum ein und entfernt Pyridinreste durch mehrmaliges Aufnehmen und Eindampfen mit Toluol. Den festen Rückstand chromatographiert man an Kieselgel (Säule 2,5 x 30 cm, Methylenchlorid/Ethanol 95:5; v:v). Man erhält zwei Zonen (von unten gezählt):

1. DC-einheitliches Tetra-O-acetyl-urdamycin G 17 mg, gelb
2. Mischfraktion, 4 mg, gelb

Man reinigt Tetra-O-acetyl-urdamycin G anschließend durch eine Chromatographie an Sephadex LH 20 (Säule 2,5 x 50 cm, Methanol) und erhält 16 mg (65 %) Tetra-O-acetyl-urdamycin G als gelben Feststoff.

Dünnschichtchromatographie (Sil G 25 $UV_{304+366}$, 20x20 cm
0,25 mm auf Glas (Macherey-Nagel), Laufstrecke 15 cm:
Chloroform/Methanol (85:15, v:v) : Rf 0,70
Chloroform/Methanol (9:1, v:v) : Rf 0,60
$C_{45}H_{54}O_{18}$ (882,8)
Schmp. 161 °C
IR (KBr): 3450; 2990; 2950; 1788; 1742; 1670; 1657 sh; 1603; 1566 $cm^{-1}$
UV (Methanol): $\lambda_{max}(\epsilon)$ = 355 (3060); 313 (3030); 259 (11760) nm
$^1H$-NMR (200 MHz, $CDCl_3$): $\delta$ = 0,51 (d, J = 6,5 Hz, 5C-$CH_3$); 1,16 (d, J = 6,5 Hz, 5A-$CH_3$); 1,26 (s, 3-$CH_3$); 1,29 (d, J = 6 Hz, 6'-$CH_3$); 1,2-2,2 (komplex, 12H, 2A-$H_2$, 3A-$H_2$, 3'-$H_2$, 4-$H_2$, 2C-$H_2$, 3C-$H_2$); 2,01; 2,11; 2,14 (3s, 5'-, 4A-und 4C-OAc); 2,49 (s, 3H, 8-OAc); 2,55 (d, J = 13 Hz, 2-$H_a$, 2,83 (dd, J = 13; 2 Hz, 2-$H_e$); 3,59 (d, J = 2 Hz OH Signal verschwindet nach $D_2O$-Austausch); 3,6-3,7 (komplex, 2H, 6'-H, 5C-H); 3,96 (dq, J = 9;6 Hz, 5A-H); 3,98 (m, 4'-H); 4,06 (s, OH * ); 4,70 (o, J = 3 x 2 Hz, 4C-H); 4,84 (o,J = 3 x 2 Hz, 4A-H); 4,86 (dd, J = 9;9 Hz, 5'-H); 5,00 (s, breit, 1A-H); 5,00 (verdeckt, 2'-H); 5,49 (d, J = 2 Hz, 1C-H); 6,42 (dd, J = 10 Hz, 5-H); 6,89 (d, J = 10 Hz, 6-H); 8,06 (d, J = 8 Hz, 11-H); 8,15 (d, J = 8 Hz, 10-H) ppm

*Signal verschwindet nach $D_2O$-Austausch

CD (Methanol):
$\lambda_{extr.}(\theta^{20}x10^{-3})$ = 501 (-4,6); 450 (1,4); 394 (-12,0); 308 (20,3); 295 sh (16,5); 266 (-11,2); 233 (25,6) nm

6. In vitro Test auf cytostatische Aktivität

Der Versuch wird nach dem Verfahren von Hamburger and Salmon [New Engl. J. Med. 298, 1321-1327 (1978)] durchgeführt. Das Medium wird durch McCoy 5A ersetzt und die Zellzahl auf $5x10^2$ Zellen/Platte reduziert.

a) Wirkung von L 1210 Leukämie-Zellen im kontinuierlichen Experiment

Es wird eine kontinuierliche Inkubation der Zellen mit verschiedenen Konzentrationen der Testsubstanz aus Beispiel 4 und 5 vorgenommen. Die zu testenden Verbindungen werden vor dem Ausplattieren der Zellkulturen auf die Agar-Platten aufgetragen. Die Zellkulturen wachsen dann 5 bis 7 Tage im Inkubator in einer Atmosphäre von 5 % $CO_2$, 20 % $O_2$ und 95 % relativer Luftfeuchte bei 37°C. Nach dieser Zeit wird die Anzahl an Zellkolonien mit einem Durchmesser ab 60 µm relativ zu der Anzahl an Zellkolonien, die im Vergleich ohne Testsubstanz gewachsen sind, bestimmt (in %).

b) Wirkung auf L 1210 Leukämie-Zellen im 1h Experiment:

Im Test werden die Zellen mit unterschiedlicher Konzentrationen der Testsubstanz 1h bei 37°C inkubiert. Danach werden die Zellen zweimal mit McCoy 5A-Lösung (Flow Katalog, Meckenheim, BRD) gewaschen und anschließend auf Agar-Platten gemäß der Methode von Hamburger und Salmon aufgetragen. Anschließend kultiviert man die Zellen wie oben beschrieben und bestimmt die Anzahl der Zellkolonien (Auswertung wie in a).

Nach der Dosis/Wirkungskurve wird der $IC_{50}$-Wert für die kontinuierliche und die 1-stündige Inkubation bestimmt.

c) Proliferations-Versuch

Tumorzellen L 1210 der exponentialen Wachstumsphase [$5x10^3$ Zellen/ml Roswell-Park-Memorial-Institute-Medium (RPMI)] werden in einer Mikrotiter Platte mit 26 Vertiefungen über einen Zeitraum von 72 Stunden bei 37°C, 5 % $CO_2$ und 95 % relativer Luftfeuchte mit verschiedenen Konzentrationen der Testsubstanzen inkubiert. Nach 65 Stunden werden 50 µl [3-(4,5-Dimethylthiazol-2,yl)-2,5-diphenyl-tetrazoliumbromid] (MTT) (2,5 mg/ml MTT in Phosphate Buffer Saline (PBS)) hinzugegeben. Das MTT wird von lebenden Zellen in den roten wasserunlöslichen Farbstoff Formazan reduziert. Nach weiteren 7 Stunden wird das überstehende Medium vorsichtig entfernt. Formazan wird durch Zugabe von 100 µl Dimethylsulfoxid pro Vertiefung und anschließendes leichtes Schütteln in Lösung gebracht. Die Extinktion jeder Vertiefung wird mit einem Multisan-Photometer 340CC, (Fa. Flow) bei 492 nm bestimmt. Die Ergebnisse werden aus dem Extinktionsverhältnis von Zellen mit Testsubstanz zu Zellen ohne Testsubstanz ermittelt.

Zu allen Fällen werden die Versuche in 4-facher Ausführung durchgeführt. Die Abweichung der Ergebnisse beträgt weniger als 15 %.

Die Ergebnisse sind in der folgenden Tabelle zusammengefaßt:

*Signal verschwindet nach $D_2O$-Austausch

| Testsubstanz | $IC_{50}$ (µl/ml) | | |
|---|---|---|---|
| | Proliferations Assay | Weichagar-Assay kontinuierliche Inkubation | 1-stündige Inkubation |
| Urdamycin G | 3,5 | 0,85 | - |
| 5',4A,4C,8-Tetra-O-acetyl-urdamycin G | < 1 | 0,036 | 0,065 |

## Ansprüche

1. Die Verbindung der allgemeinen Formel I,

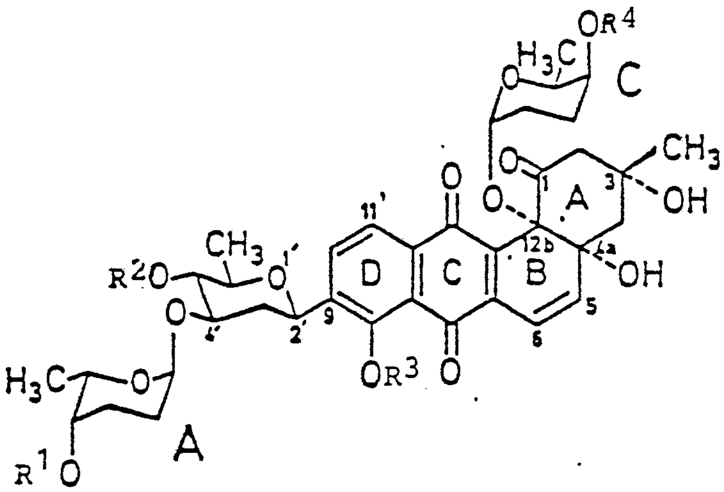

I

in der $R^1$ bis $R^4$ unabhängig voneinander Wasserstoff oder eine ($C_1$-$C_{18}$)-Acylgruppe bedeutet.

2. Die Verbindung nach Anspruch 1, in der $R^1$ bis $R^4$ unabhängig voneinander Wasserstoff oder ($C_1$-$C_{10}$)-Acyl bedeutet.

3. Die Verbindung nach Anspruch 1, in der $R^1$ bis $R^4$ unabhängig voneinander Wasserstoff oder Acetyl bedeutet.

4. Das Verfahren zur Herstellung der Verbindung der allgemeinen Formel I,

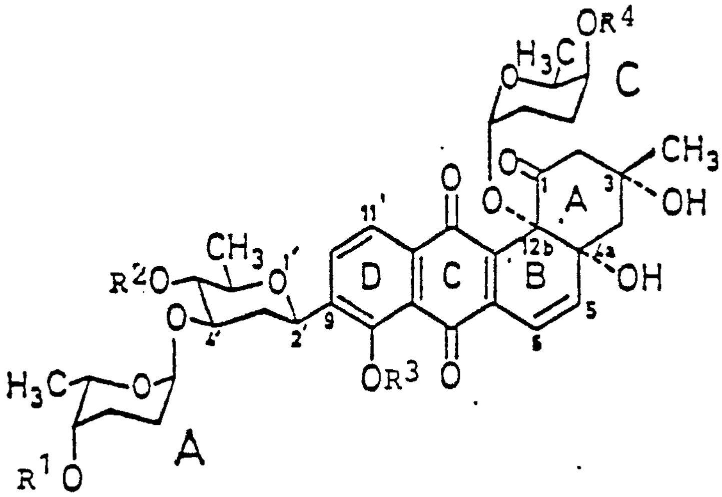

I

in der $R^1$ bis $R^4$ unabhängig voneinander Wasserstoff oder eine ($C_1$-$C_{18}$)-Acylgruppe bedeutet, dadurch gekennzeichnet, daß man

a) Streptomyces fradiae kultiviert

b) die Verbindung der allgemeinen Formel I, in der $R^1$ bis $R^4$ Wasserstoff bedeutet isoliert und gegebenenfalls

c) diese Verbindung acyliert.

5. Verfahren nach Anspruch 4, dadurch gekennzeichnet, daß Streptomyces fradiae DSM 3093 eingesetzt wird.

6. Verfahren nach Anspruch 4 oder 5, dadurch gekennzeichnet, daß Streptomyces fradiae in einer Nährlösung mit 0,5 bis 5 % Vollfett-Soja kultiviert wird.

7. Verfahren nach einem oder mehreren der Ansprüche 4 bis 6, dadurch gekennzeichnet, daß Streptomyces fradiae in einem Temperaturbereich von 18 bis 40°C kultiviert wird.

8. Verfahren nach Anspruch 7, dadurch gekennzeichnet, daß der Temperaturbereich 25 bis 30°C beträgt.

9. Verfahren nach einem oder mehreren der Ansprüche 4 bis 8, dadurch gekennzeichnet, daß Streptomyces fradiae über einen Zeitraum von 40 bis 80 Stunden kultiviert wird.

10. Verfahren nach Anspruch 9, dadurch gekennzeichnet, daß Streptomyces fradiae 70 bis 75 Stunden kultiviert wird.

11. Verwendung der Verbindung der allgemeinen Formel I nach Anspruch 1 bis 3 zur Herstellung von Heilmitteln.

12. Verwendung nach Anspruch 11 zur Herstellung eines Heilmittels zur Behandlung von Tumor-und infektiösen Erkrankungen.

13. Verwendung der Verbindung der allgemeinen Formel I nach Anspruch 1 bis 3 als antibiotisches Agens.

Patentansprüche für die folgenden Vertragsstaaten: GR, AT und ES:

1. Das Verfahren zur Herstellung der Verbindung der allgemeinen Formel I,

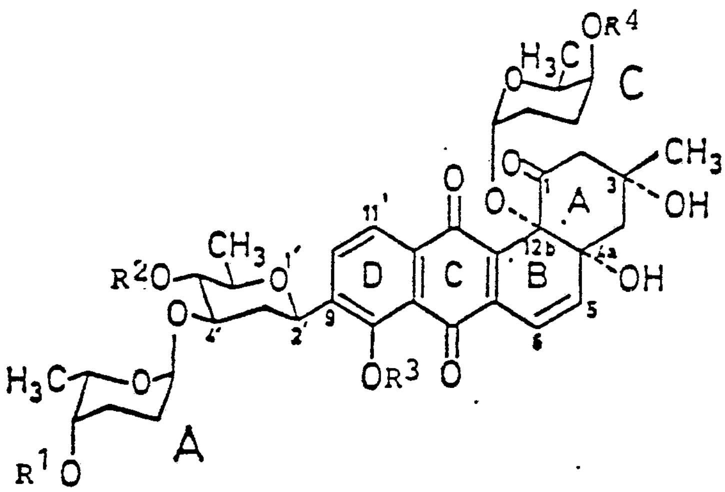

I

in der $R^1$ bis $R^4$ unabhängig voneinander Wasserstoff oder eine ($C_1$-$C_{18}$)-Acylgruppe bedeutet, dadurch gekennzeichnet, daß man

a) Streptomyces fradiae kultiviert

b) die Verbindung der allgemeinen Formel I, in der $R^1$ bis $R^4$ Wasserstoff bedeutet isoliert und gegebenenfalls

c) diese Verbindung acyliert.

2. Verfahren nach Anspruch 1, dadurch gekennzeichnet, daß Streptomyces fradiae DSM 3093 eingesetzt wird.

3. Verfahren nach Anspruch 1 oder 2, dadurch gekennzeichnet, daß Streptomyces fradiae in einer Nährlösung mit 0,5 bis 5 % Vollfett-Soja kultiviert wird.

4. Verfahren nach einem oder mehreren der Ansprüche 1 bis 3, dadurch gekennzeichnet, daß Streptomyces fradiae in einem Temperaturbereich von 18 bis 40°C kultiviert wird.

5. Verfahren nach Anspruch 4, dadurch gekennzeichnet, daß der Temperaturbereich 25 bis 30°C beträgt.

6. Verfahren nach einem oder mehreren der Ansprüche 1 bis 5, dadurch gekennzeichnet, daß Streptomyces fradiae über einen Zeitraum von 40 bis 80 Stunden kultiviert wird.

7. Verfahren nach Anspruch 6, dadurch gekennzeichnet, daß Streptomyces fradiae 70 bis 75 Stunden kultiviert wird.

8. Verwendung der Verbindung der allgemeinen Formel I erhältlich nach Anspruch 1 bis 7 zur Herstellung von Heilmitteln.

9. Verwendung nach Anspruch 8 zur Herstellung von Heilmitteln zur Bekämpfung von Tumor-oder infektiösen Erkrankungen.

10. Verwendung der Verbindung der allgemeinen Formel I erhältlich nach Anspruch 1 bis 7 als antibiotisches Agens.